# FASCICULE DE BREVET EUROPEEN

(11) **EP 0 552 576 B1**
(45) Date de publication et mention de la délivrance du brevet: **25.02.1998**
(21) Numéro de dépôt: 92403501.7
(22) Date de dépôt: 21.12.1992
(51) Int. Cl.: C08L 89/06, C08H 1/06, A61L 15/00

(54) **Procédé de préparation de fibres de collagène**
Verfahren zur Herstellung von Kollagenfasern
Process for producing collagen fibres

(30) Priorité: 24.01.1992 FR 9200739
(43) Date de publication de la demande: 28.07.1993
(73) Titulaire: LABORATOIRES FOURNIER S.A., 21100 Dijon (FR)
(72) Inventeur: Janod, Philippe, F-39100 Dole (FR)
(74) Mandataire: Clisci, Serge

(56) Documents cités:
- EP-A- 0 331 786
- DE-A- 1 913 563
- FR-A- 1 568 829
- FR-A- 2 200 377
- FR-A- 2 371 475
- DATABASE WPIL Section Ch, Week 8251, Derwent Publications Ltd., London, GB; Class A, AN 82-11219J & SU-A-908 357

## Description

La présente invention concerne une nouveau procédé de préparation de fibres de collagène présentant un haut pouvoir hémostatique.

Le collagène est une protéine très abondante dans les tissus conjonctifs des mammifères. Il constitue notamment le composant principal de la peau. C'est ainsi que les peaux d'animaux représentent une source importante de collagène.

La technique d'extraction du collagène à partir des peaux d'animaux est bien connue depuis de longues années. Elle consiste à préparer les peaux en éliminant les matières indésirables comme les poils, l'épiderme et la chair par des traitements physiques, comme par exemple le rinçage à l'eau, des traitements mécaniques, comme par exemple l'écharnage et le refandage et des traitements chimiques, comme par exemple le chaulage par un mélange de chaux et de sulfure de sodium, puis déchaulage par un mélange de chlorure d'ammonium et de métabisulfite de sodium et enfin extraction du collagène par un acide organique, de préférence un acide organique chloré comme préconisé par le brevet français FR-A-1568 829.

Afin de conserver d'une part la structure hélicoïdale, d'autre part la structure moléculaire du collagène natif, les traitements physiques et chimiques doivent être effectués à une température ne dépassant pas 30 à 32°C comme préconisé par FR-A-1 568 829 et dans des conditions d'alcalinité ou d'acidité contrôlées.

On sait en effet par la demande de brevet français FR-A-2 371 475 que les télopeptides du collagène se trouvant aux extrémités des chaînes polypeptidiques sont hydrolysés par les substances alcalines, notamment lors d'un traitement par la soude à une concentration de 0,3 à 1N pendant 5 heures à 10 jours, la structure moléculaire du collagène se trouvant ainsi modifiée. On sait également par la demande de brevet européen EP-A-0 081 440 qu'un traitement alcalin prolongé, par exemple à pH 14 pendant 8 jours, conduit à une déréticulation du collagène, c'est-à-dire à la destruction de la structure hélicoïdale du collagène.

On vient à présent de trouver qu'un traitement du collagène en milieu basique avec une base forte, à une concentration de 1N, pendant 0,5 à 1,5 heure et à une température ne dépassant pas 32°C, non seulement ne modifie pas la structure hélicoïdale du collagène ni sa structure moléculaire, mais permet d'obtenir des fibres de collagène natif qui présentent, de façon inattendue, un pouvoir hémostatique supérieur au pouvoir hémostatique des fibres de collagène obtenues par les procédés connus de l'art antérieur.

Le procédé de préparation de gels et de fibres de collagène natif à partir de peaux animales selon l'invention consiste à éliminer les substances non collagéniques, procéder à un traitement chimique alcalin par une base forte, à une concentration de 1N, à une température ne dépassant pas 32°C, pendant 0,5 à 1,5 heure, puis neutraliser le milieu, extraire le collagène par un acide organique, de préférence un acide organique chloré et le cas échéant pour obtenir les fibres de collagène, lyophiliser le gel obtenu après dilution et purification, ledit gel lyophilisé étant constitué de fibres de collagène.

Plus précisément, on vise un procédé de préparation d'un matériau choisi parmi les gels et les fibres de collagène natif à partir de peaux animales, selon lequel on élimine les substances non collagéniques, procède à un traitement chimique alcalin, neutralise le milieu, extrait le collagène par un acide organique et, le cas échéant, lyophilise le gel obtenu après dilution et purification, caractérisé en ce que le traitement chimique alcalin consiste en l'action d'une base forte, à une concentration de 1N, à une température ne dépassant pas 32°C, pendant 0,5 à 1,5 heure.

De façon avantageuse le traitement chimique alcalin consiste en l'action de la soude ou la potasse à une concentration de 1N, à la température ambiante (18-25°C), pendant 1 heure.

L'invention englobe également les gels et fibres de collagène natif obtenus selon ledit procédé.

Les propriétés hémostatiques du collagène sont bien connues. Cependant, de façon surprenante, on a observé que les fibres de collagène natif obtenues par le procédé selon l'invention possèdent un pouvoir hémostatique très nettement supérieur au pouvoir hémostatique des fibres de collagène obtenues par les procédés connus de l'art antérieur.

On a préparé 2 lots de fibres de collagène par le procédé selon l'invention, c'est-à-dire par traitement du collagène par la soude 1N pendant une heure à 20°C, et on a déterminé leur pouvoir hémostatique par comparaison avec un produit de l'art antérieur constitué de collagène natif préparé selon un procédé ne différant de celui de l'invention que par l'absence de l'étape de traitement alcalin et commercialisé sous la dénomination PANGEN par la société Laboratoires FOURNIER.

Le pouvoir hémostatique des différents produits a été évalué in vitro selon la méthode décrite par Rosy Eloy et al. dans Journal of Biomedical Materials Research, Vol.22, 149-157 (1988), sur sang humain complet non anticoagulé de 3 donneurs différents. L'activation de l'hémostase au contact de 50 mg (sauf indication contraire dans le tableau I suivant) de chacun des produits divisés en 12 parties égales est évaluée par mesure de la cinétique de génération du Fibrinopeptide A. La comparaison des différents produits entre eux est réalisée par la méthode des scores attribués selon le rang d'activité de chacun des produits (de 3 pour le produit le plus actif à 0 pour le produit le moins actif) pour chaque donneur et à des temps de prélèvement correspondant aux 3^{ème}, 4^{ème}, 5^{ème} et 6^{ème} minutes.

Les scores totaux obtenus sont regroupés dans le tableau I suivant :

**Tableau I**

| **fibres de collagène selon l'invention** | **score total** |
|---|---|
| lot 1 | 27 |
| lot 2 | 21 |
| | |

| **produits comparatifs** | **score total** |
|---|---|
| PANGEN | 14 |
| PANGEN (1) | 11 |

| | |
|---|---|
| note (1) : 25 mg de collagène | |

On a vérifié que le temps de coagulation d'un sang humain complet, en l'absence de fibres de collagène, est trois fois plus long qu'en présence du collagène des produits comparatifs.

Il ressort de ces résultats que les fibres de collagène obtenues par le procédé selon l'invention accélèrent la cinétique de génération du Fibrinopeptide A et présentent un pouvoir hémostatique 1,5 à 2,5 fois supérieur à des fibres de collagène obtenues par un procédé ne différant de celui de l'invention que par l'absence de l'étape de traitement alcalin.

Le procédé selon l'invention est applicable à la fabrication de gels de collagène et de fibres de collagène, notamment sous forme de compresses, utilisables dans le domaine des pansements et en chirurgie comme agents hémostatiques de contact et pour le contrôle des hémorragies.

## Revendications

1. Procédé de préparation d'un matériau choisi parmi les gels et les fibres de collagène natif à partir de peaux animales, selon lequel on élimine les substances non collagéniques, procède à un traitement chimique alcalin, neutralise le milieu, extrait le collagène par un acide organique et, le cas échéant, lyophilise le gel obtenu après dilution et purification, caractérisé en ce que le traitement chimique alcalin consiste en l'action d'une base forte, à une concentration de 1N, à une température ne dépassant pas 32°C, pendant 0,5 à 1,5 heure.

2. Procédé selon la revendication 1, caractérisé en ce que la base forte est la soude.

3. Procédé selon la revendication 1, caractérisé en ce que la base forte est la potasse.

4. Procédé selon l'une quelconque des revendications 1 à 3, caractérisé en ce que le traitement chimique alcalin est réalisé à une température comprise entre 18 et 25°C.

5. Procédé selon l'une quelconque des revendications 1 à 4, caractérisé en ce que la durée du traitement alcalin est de 1 heure.

## Patentansprüche

1. Verfahren zur Herstellung eines Materials, das aus den Gelen und den Fasern von natürlichem Kollagen ausgewählt ist, ausgehend von Tierhäuten, wobei man die nicht-kollagenen Substanzen eliminiert, eine alkalische chemische Behandlung anschließt, das Milieu neutralisiert, das Kollagen durch eine organische Säure extrahiert und das nach Verdünnung und Reinigung erhaltene Gel gegebenenfalls lyophilisiert,
dadurch **gekennzeichnet,** daß die alkalische chemische Behandlung aus der Einwirkung einer starken Base, bei einer Konzentration von 1 N, bei einer Temperatur nicht über 32°C, während 0,5 bis 1,5 Stunden besteht.

2. Verfahren nach Anspruch 1,
dadurch **gekennzeichnet,** daß die starke Base Natronlauge ist.

3. Verfahren nach Anspruch 1,
dadurch **gekennzeichnet,** daß die starke Base Kaliumlauge ist.

4. Verfahren nach einem der Ansprüche 1 bis 3,
dadurch **gekennzeichnet,** daß die alkalische chemische Behandlung bei einer Temperatur zwischen 18 und 25°C durchgeführt wird.

5. Verfahren nach einem der Ansprüche 1 bis 4,
dadurch **gekennzeichnet,** daß die Dauer der alkalischen Behandlung eine Stunde beträgt.

## Claims

1. A method for preparing a collagen product, which is selected from gels and fibers of native collagen, from animal skins, said method, which comprises removing not collageneous materials, an alkaline chemical treatment, neutralizing the reaction medium, then extracting collagen with an organic acid, and if required freeze-drying the gel obtained after dilution and purification, being characterized in that the alkaline chemical treatment consists in performing with an alkaline strong base, at a base concentration of about 1N, at a temperature not exceeding 32° C, and for a duration offrom 0.5 to 1.5 h.

2. A method according to claim 1, in which said strong base is NaOH.

3. A method according to claim 1, in which said strong base is KOH.

4. A method according to any one of claims 1-3, in which said alkaline treatment is carried out at a temperature of between 18 and 25°C.

5. A method according to any one of claims 1-4, in which said alkaline treatment has a duration of 1 hour.
